# EUROPEAN PATENT SPECIFICATION

(11) **EP 1 334 096 B1**
(45) Date of publication and mention of the grant of the patent: **28.07.2004**
(21) Application number: 00974698.3
(22) Date of filing: 15.11.2000
(51) Int. Cl.: C07D 335/16

(54) **FRIEDEL-CRAFT PROCESS FOR THE PREPARATION OF THIOXANTHONES**
FRIEDEL-CRAFT VERFAHREN ZUR HERSTELLUNG VON THIOXANTHONEN
PROCEDE FRIEDEL-CRAFT POUR LA PREPARATION DE THIOXANTHONES

(43) Date of publication of application: 13.08.2003
(73) Proprietor: Prom Limited, Caterham CR3 5UH (GB)
(72) Inventor: BERG, Carsten, DK-4791 Borre (DK)
(74) Representative: Gambell, Derek
(86) International application number: PCT/GB2000/004343
(87) International publication number: WO 2002/040464

(56) References cited:
- GB-A- 2 050 378
- US-A- 4 101 558

## Description

The invention relates to a process for the preparation of thioxanthen-9-one derivatives, of the following thioxanthones, of the Formula 1.

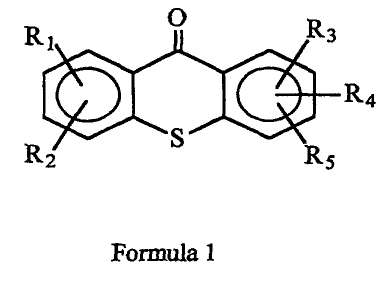

Thioxanthones are useful intermediates for the preparation of pharmaceuticals in the field of psychoterapeutics.

Another important use is as activators or sensitizers in the photopolymerisation of ethylenically unsaturated monomers.

Basically thioxanthones are formed by cyclisation of 2-phenylthiobenzoic acid derivatives. As described by Smiles, J. Chem. Soc. (1911), 99, 645, this reaction can be performed in one step, by reacting 2,2'-dithiodibenzoic acid with an aromatic compound in sulfuric acid. This invention has been the most utilised for producing thioxanthones in an industrial scale.

The main drawbacks of the process is that yields are poor, 40 - 60%, a large excess of sulfuric acid must be used, after reaction a large amount of diluted sulfuric acid has to be regenerated or disposed. Sulfonated aromatics are major biproducts.

For that reason 2-chlorothiobenzoyl chloride (CTBC) or derivatives hereof has been considered as an obvious choice for a Friedel-Craft type reaction to form thioxanthones. This reaction is first revealed in patent US4101558 where 2-chlorothiobenzoyl - and 5-chloro-2-chlorobenzoyl chloride is reacted with benzene and chlorobenzene to form thioxanthone, 7-chlorothioxanthone and 2,7-dichlorothioxanthone with aluminium chloride as catalyst.

In the examples of preparation, CTBC and the aromatic substrate are mixed in a suitable solvent and aluminium chloride is added in small portions giving rise to product yields from 70 - 91 %. The patent claims do not specify in which order the Friedel-Craft catalyst and the two reactants are brought together.

Belleau et aL, Synth. Commun., (1983), 13, 977 - 984, have investigated different types of Lewis acids as catalyst to form thioxanthones from CTBC and ortho-/para-xylene or 1,4-dimethoxy substituted aromatic substrates. They found that in this case of activated aromatic substrates tin(IV)chloride was the most effective catalyst.

The experimental section describes that CTBC in a chlorinated solvent is mixed with tin(IV) chloride, where after the substrate is added in one portion.

### Disclosure of invention

It is an object of this invention to provide a facile process for the preparation of thioxanthones, specially 2-isopropyl- and 2-chlorothioxanthone in commercial quantities, good yield and high purity.

To avoid the problems connected to the method using sulfuric acid as medium for reaction of 2,2'-dithiodibenzoic acid with an aromatic substrate, the reaction between CTBC and an aromatic substrate under Friedel-Craft conditions were pursued according to Reaction Scheme1 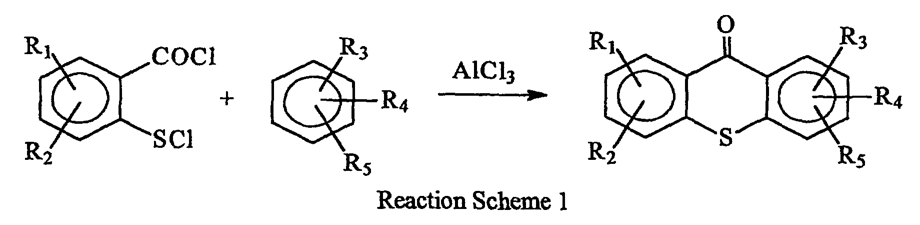 CTBC is prepared by known methods where 2,2'-dithiodibenzoic acid or substituted derivatives hereof is transformed to the acid chloride by reaction with thionyl chloride or a similar reagent. The reaction is facilitated by using thionyl chloride as solvent and addition of a catalyst of the amide type like dimethylformamide or N-methyl-2-pyrrolidinon. The acid chloride is chlorinated with chlorine or sulfuryl chloride revealing CTBC.

As CTBC where R₁ = R₂ = H also is an important starting material for the industrial production of the biocide, 1,2-benzisothiazolin-3-one, investigations were done for purification of CTBC. Even the literature does not disclose examples of distilling the product, it was shown that this could be done by a vacuum distillation at, 165 - 170°C and 1333 Pa. (10 mm Hg), giving a light yellow product with m.p. 67 -68°C, from the brown crude starting material. CTBC was found to be heat sensitive by prolonged heating to about 200°C. To avoid decomposition in industrial scale, distillation should preferably be performed under mild conditions like falling- or wiped film evaporations.

Experiments with CTBC wherein R₁ = R₂ = H and substrate isopropylbenzene (cumene) wherein R₃ = i-C₃H₇, R₄ = R₅ = H in 1,2-dichloroethane (DCE) as solvent and aluminium chloride as catalyst, were performed according to the conditions in US4101558.

The content of isomers 2- and 4-isopropylthioxanthone in the organic phase were found to be 70% of theory, by HPLC, and 50% as isolated yield.

The reaction could not be performed as described by Belleau et al. as CTBC disproportionated with aluminium chloride in DCE.

Using the substrate, cumene, as solvent with aluminium chloride and adding CTBC to the slurry gave rise to formation of an insoluble compound, which probably is a reaction product from 1 mole CTBC with 2 moles cumene.

Surprisingly it turned out that the reaction could be performed if a mixture of CTBC and cumene, with a small molar excess of cumene, in DCE, were added to a slurry of aluminium chloride, in small molar excess, and DCE. After water quench the total yield of 2- and 4-thioxanthone isomers in the organic phase were 89% of theory, by calibrated HPLC.

By performing the process in the described way handling aluminium chloride in industrial scale has been facilitated, as the catalyst has not to be added during the process.

The process has been extended to thioxanthones, according to Reaction Scheme 1, where R₁ is different from, or equals R₂ both are represented by hydrogen, chlorine, bromine, alkyl, aryl and alkoxy radicals having 1 to 8 carbon atoms. R₃, R₄ and R₅ can equals one another by pair or all three, or be different and be represented by hydrogen, chlorine, bromine, hydroxyl, alkyl, cycloalkyl, aryl, alkoxy, alkylcarbonylalkoxy, carbonylalkyl, carbonylalkoxy, carbamido or R₃ and R₄ can form a 5, 6 or seven member ring fused to the aromatic ring.

R₃, R₄ and R₅ can be radicals having 1 to 10 carbon atoms. Specific types of substituted thioxanthone are 2-chloro, 4-chloro, 2-bromo, 4-bromo, 2,7-dichloro, 2-hydroxy, 2-methyl, 4-methyl, 2,4-diethyl, 2-isopropyl, 2-carboethoxymethyl. The aromatic compound may include polynuclear aromatic compounds as biphenyl as naphthalene.

The process is carried out in an organic solvent which are particularly known to be useful for Friedel-Craft reaction such as methylene chloride, 1,2-dichloroethane, 1,1,2,2-tetrachloroethane, etc.

To perform the reaction, known Friedel-Craft catalyst as aluminium chloride, aluminium bromide, iron (III) chloride, tin(II) chloride, tin(IV) chloride, iron(III) chloride can be used. Specially aluminium chloride.

The reaction may preferably be carried out using substitutet or unsubstitutet CTBC : aromatic substrate : Friedel-Craft catalyst in the molar ratio 1 : 1 : 1 - 1 : 20 : 5. The molar ratio of said components is particularly advantageously as 1 : 1,2 : 1,6.

The reaction may be performed at a temperature between 0°C and 80°C.

Preferably 25°C.

The method of isolating the thioxanthones from the organic reaction media may vary depending on the physical and chemical nature of the product. If the complex between the product and aluminium chloride is sparingly soluble, it can be an advantage to isolate the complex before decomposition. Preferably decomposition of the complex can be accomplished by adding the reaction mixture to water, diluted mineral acid or aqueous solutions of alkaline hydroxides like sodium, potassium, barium or calcium. The choice of decomposition medium depends on the base or acid nature of the product and of insoluble by-products, keeping the product in the organic phase and by-products in the water phase. Crude thioxanthones are isolated by evaporation of the solvent, and if necessary purified by methods as crystallisation, chromatographi etc.

### Examples

Reaction mixtures and products were analysed by HPLC chromatographi.

Specific compounds were characterised by comparison with authentic samples.

### HPLC conditions:

| | | |
|---|---|---|
| Column | : | Merck, LiChrospher 100-RP 18, 5 micron 250 x 4 mm |
| Detector wavel. | : | 254 nm |
| Injection Vol. | : | 20 microL |
| Flow rate | : | 2 mL / min |
| Eluents: | : | A: 0.005 M H₃PO₄ in Water; B: Acetonitrile |
| Gradient | : | Time 0, %B 40; 10, 90; 15, 90; 20, 40. |
| Isocratic | : | %B 66 |

### Example 1

### 2-Chlorothiobenzoyl chloride (CTBC)

2,2'-dthiodibenzoic acid, assay 95%, 100 g, 03105 moles, and 1-methyl-2-pyrrolidinone, 1.0 g, 0.010 moles, was added to thionyl chloride 260 g, 2.185 moles. The stirred slurry was heated slowly to reflux, 80°C, until it has become a clear solution and HCl evolution had ceased. The mixture was cooled to 50°C and sulfuryl chloride, 48.6 g, 03600 moles, was slowly introduced. Stirring was continued for 30 min, where after volatiles were evaporated off on a rotary evaporator leaving crude, CTBC, 140.0 g, as a light brown solid. The product was vacuum distilled through a short Vigreux column at 165-170°C, 1333 Pa (10 mmHg), affording CTBC, 119.5g, 0.5773 moles, as a light yellow crystalline product in 93% yield oftheory.

Purity was estimated to 98.5 % by derivatisation with ammonia to 1,2-benzisothiazolin-3-one and running HPLC of the solution.

### Example 2

### 2-/4-isopropylthioxanthone (ITX)

Crude CTBC, 95% assay, 7.6 g, 0.0349 moles, and cumene, 4.6 g, 0.0383 moles, were dissolved in 1,2-dichloroethane (DCE), 70 g. This solution was added over 45 min to a stirred slurry of aluminium chloride, 7.3 g, 0.0548, in DCE, 50 g covered with nitrogen, with temperature kept at 20 - 25°C. Temperature was raised to 30°C for 30 min, where after the reaction mixture was poured into 5% hydrochloric acid, 100 mL, with vigorous stirring. The organic phase was separated and washed once with water. HPLC on the organic phase revealed a content of 7.5 g, 0.0295 moles ITX. Corresponding to 85% yield of theory. Volatiles were evaporated off in vacuo leaving crude ITX as an orange oil, 9.44 g, which was crystallised from methanol, 28 g, leaving ITX, 6.11 g, 0.0240 moles, 69% yield of theory, as light yellow crystals, with purity, by HPLC, 97.3% and m.p. 73 - 74°C.

### Example 3

### ITX from distilled CTBC

The experiment in Example 2 was repeated using distilled CTBC.

Results were nearly similar, except for purity, which was estimated, by HPLC, to 99.2%.

### Example 4

### 2-/4-chlorothioxanthone (CTX)

Crude CTBC, 95 % assay, 0.0481 moles, and chlorobenzene, 6.7 g, 0.0588 moles, dissolved in DCE, 16 g, was added over 30 min to a stirred slurry of aluminium chloride in DCE, 150 g, covered with nitrogen, keeping temperature at 40 -45°C. The reaction mixture was stirred for a further 30 min at 40°C and poured into stirred water keeping pH at 12 with 50 w/w% sodium hydroxide.

The DCE phase was separated and washed once with water. HPLC run revealed a content of 11.1 g, 0.0450 moles of CTX corresponding to 94% yield of theory. The solvent was evaporated on rotary evaporator to a weight of 20.1 g. Separated crystals was isolated, washed with a little ethanol and dried affording CTX, 8.6 g, 0.0349 moles, with purity, by HPLC, 98.8% and m.p. 143 - 145°C. Yield of theory was 73%.

## Claims

1. A process for the preparation of one or more thioxanthones represented by Formula 1: 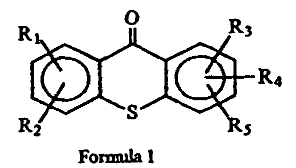 wherein R¹ and R² are the same or different and are selected from hydrogen, chlorine, bromine, and alkyl, aryl and alkoxy radicals having from 1 to 8 carbon atoms, and R³, R⁴ and R⁵ are the same or different and are selected from hydrogen, chlorine, bromine, hydroxy, and alkyl, alkoxy, alkylcarbonylalkoxy, carbonylalkyl, carbonylalkoxy, or carbamido radicals having from 1 to 10 carbon atoms, or R³ and R⁴ together form a 5,6 or 7 member ring fused to the aromatic ring,
which process comprises adding a reactant mixture of a 2-chlorothiobenzoyl chloride (A) represented by Formula 2: 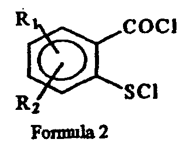 and an aromatic compound (B) represented by Formula 3: 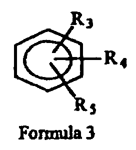 to a slurry of a Friedel-Crafts catalyst in an organic Friedel-Crafts solvent.

2. The process of claim 1 wherein the Friedel-Crafts catalyst is selected from aluminium chloride, aluminium bromide, iron (III) chloride, tin (II) chloride, tin (IV) chloride and iron (III) chloride, especially aluminium chloride.

3. The process of claim 1 or 2, wherein the Friedel-Crafts catalyst is used in an amount of 1.0 to 2.5, such as from 1.1 to 1.6, moles per mole of 2-chlorothiobenzoyl chloride (A).

4. The process according to any preceding claim, wherein R¹ and R² are both hydrogen.

5. The process according to any preceding claim, wherein R³ is isopropyl and R⁴ and R⁵ are both hydrogen.

6. The process according to any preceding claim, wherein the Friedel-Crafts solvent is selected from methylene chloride, 1,2-dichloroethane and 1,1,2,2-tetrachloroethane.

7. The process according to any preceding claim, wherein the Friedel-Crafts solvent is additionally present in the reactant mixture before the latter is added to the slurry.

8. The process according to claim 7, wherein the reactants are dissolved in a Friedel-Crafts solvent, in an amount of 1 to 30 parts by weight per part by weight of the reactant mixture.

9. The process according to any preceding claim, wherein the slurry contains 1 to 20 parts by weight of the Friedel-Crafts solvent per part by weight of the catalyst.

10. The process according to any preceding claim, wherein the reactant mixture contains from 1 to 20, especially from 1.1 to 1.2, moles of the aromatic compound (B) per mole of the 2-chlorothiobenzoyl chloride (A).

11. The process according to any preceding claim, carried out at a temperature of from 0° to 80°C, especially from 20°C to 45°C.

12. The process according to any preceding claim, wherein the 2-chlorothiobenzoyl chloride (A) has previously been purified by vacuum distillation at a temperature of from 80°C to 200°C, and a pressure of from 13,3 to 39 997 Pa (0.1 to 300 mm Hg).

13. One or more thioxanthones prepared by a process according to any preceding claim.

14. A crystalline mixture of 2- and 4- isopropyl thioxanthone having a melting point of about 73°C to 74°C.

15. A crystalline mixture of 2- and 4- chloro thioxanthone having a melting point of about 143°C to 145°C.

16. The use of one or more thioxanthones according to any one of claims 12 to 14, for the preparation of a pharmaceutical product for use in the field of psychotherapeutics, or as activators or sensitizers in the photopolymerisation of ethylenically unsaturated monomers.

## Patentansprüche

1. Verfahren zur Herstellung von einem oder mehreren Thioxanthonen der Formel 1: 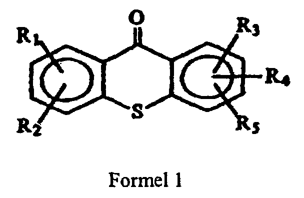 worin R¹ und R² gleich oder verschieden sind und ausgewählt sind unter Wasserstoff, Chlor, Brom, und Alkyl, Aryl und Alkoxyresten mit 1 bis 8 Kohlenstoffatomen, und R³, R⁴ und R⁵ gleich oder verschieden sind und ausgewählt sind unter Wasserstoff, Chlor, Brom, Hydroxy, und Alkyl, Alkoxy, Alkylcarbonylalkoxy, Carbonylalkyl, Carbonylalkoxy oder Carbamidoresten mit 1 bis 10 Kohlenstoffatomen, oder R³ und R⁴ zusammen einen 5-, 6- oder 7-gliedrigen Ring bilden, der an den aromatischen Ring kondensiert ist,
bei welchem Verfahren man eine Reaktantenmischung aus einem 2-Chlorothiobenzoylchlorid (A) der Formel 2: 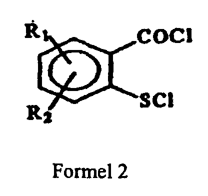 und eine aromatische Verbindung (B) der Formel 3: 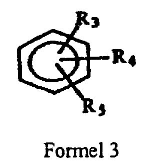 zu einer Aufschlämmung eines Friedel-Crafts Katalysators in einem organischen Friedel-Crafts Lösungsmittel hinzufügt.

2. Verfahren gemäß Anspruch 1, **dadurch gekennzeichnet, dass** der Friedel-Crafts Katalysator ausgewählt ist aus Aluminiumchlorid, Aluminiumbromid, Eisen(III)-Chlorid, Zinn(II)-Chlorid, Zinn(IV)-Chlorid und Eisen(III)-Chlorid, insbesonders Aluminiumchlorid.

3. Verfahren nach Anspruch 1 oder 2, **dadurch gekennzeichnet, dass** der Friedel-Crafts Katalysator in einer Menge von 1,0 bis 2,5, wie zum Beispiel von 1,1 bis 1,6, Molen pro Mol 2-Chlorothiobenzoylchlorid (A) eingesetzt wird.

4. Verfahren nach einem der vorhergehenden Ansprüche, **dadurch gekennzeichnet, dass** R¹ und R² beide Wasserstoff sind.

5. Verfahren gemäß einem der vorhergehenden Ansprüche, **dadurch gekennzeichnet, dass** R³ Isopropyl ist und R⁴ und R⁵ beide Wasserstoff sind.

6. Verfahren gemäß einem der vorhergehenden Ansprüche, **dadurch gekennzeichnet, dass** das Friedel-Crafts Lösungsmittel ausgewählt ist aus Methylenchlorid, 1,2-Dichlorethan und 1,1,2,2-Tetrachlorethan.

7. Verfahren gemäß einem der vorhergehenden Ansprüche, **dadurch gekennzeichnet, dass** das Friedel-Crafts Lösungsmittel zusätzlich in der Reaktantenmischung vorhanden ist bevor letztere zu der Aufschlämmung zugefügt wird.

8. Verfahren gemäß Anspruch 7, **dadurch gekennzeichnet, dass** die Reaktanten in einem Friedel-Crafts Lösungsmittel gelöst werden, in einer Menge von 1 bis 30 Gewichtsteilen pro Gewichtsteil der Reaktantenmischung.

9. Verfahren gemäß einem der vorhergehenden Ansprüche, **dadurch gekennzeichnet, dass** die Aufschlämmung 1 bis 20 Gewichtsteile des Friedel-Crafts Lösungsmittels pro Gewichtsteil des Katalysators enthält.

10. Verfahren gemäß einem der vorhergehenden Ansprüche, **dadurch gekennzeichnet, dass** die Reaktantenmischung 1 bis 20, insbesonders 1,1 bis 1,2, Mole der aromatischen Verbindung (B) pro Mol des 2-Chlorothiobenzoylchlorids (A) enthält.

11. Verfahren gemäß einem der vorhergehenden Ansprüche, **dadurch gekennzeichnet, dass** man es bei einer Temperatur von 0° bis 80°C, insbesonders von 20°C bis 45°C, ausführt.

12. Verfahren gemäß einem der vorhergehenden Ansprüche, **dadurch gekennzeichnet, dass** das 2-Chlorothiobenzoylchlorid (A) zuvor durch Vakuumdestillation bei einer Temperatur 80°C bis 200°C und einem Druck von 13,3 bis 39.997 Pa (0,1 bis 300 mm Hg) gereinigt worden ist.

13. Ein oder mehrere Thioxanthone hergestellt gemäß einem Verfahren nach den vorhergehenden Ansprüchen.

14. Eine kristalline Mischung aus 2- und 4-Isopropylthioxanthon mit einem Schmelzpunkt von etwa 73°C bis 74°C.

15. Kristalline Mischung von 2- und 4-Chlorothioxanthon mit einem Schmelzpunkt von etwa 143°C bis 145°C.

16. Verwendung von einem oder mehreren Thioxanthonen gemäß einem der Ansprüche 12 bis 14, zur Herstellung eines pharmazeutischen Produkts zur Verwendung auf dem Gebiet der Psychotherapeutika oder als Aktivatoren oder Sensibilisatoren in der Photopolymerisation von ethylenisch ungesättigten Monomeren.

## Revendications

1. Procédé de préparation d'une ou de plusieurs thioxanthones représentées par la formule 1 : 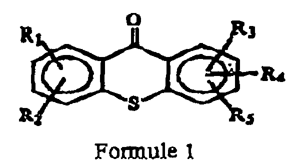 dans laquelle R¹ et R² sont identiques ou différents et sont choisis parmi les atomes d'hydrogène, de chlore, de brome, et les radicaux alkyle, aryle et alcoxy comportant de 1 à 8 atomes de carbone, et R³, R⁴ et R⁵ sont identiques ou différents et sont choisis parmi les atomes d'hydrogène, de chlore, de brome, et les radicaux hydroxy, alkyle, alcoxy, alkylcarbonylalcoxy, carbonylalkyle, carbonylalcoxy ou carbamido comportant de 1 à 10 atomes de carbone, ou R³ et R⁴ forment ensemble un noyau à 5, 6 ou 7 chaînons condensé au noyau aromatique,
ce procédé comprenant le fait d'ajouter un mélange réactionnel d'un chlorure de 2-chlorothiobenzoyle (A) représenté par la formule 2 : 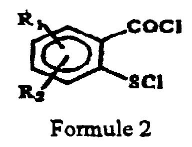 et d'un composé aromatique (B) représenté par la formule 3 : 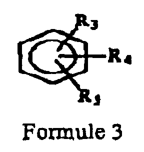 à une suspension d'un catalyseur de Friedel et Crafts dans un solvant organique de Friedel et Crafts.

2. Procédé selon la revendication 1 dans lequel le catalyseur de Friedel et Crafts est choisi parmi le chlorure d'aluminium, le bromure d'aluminium, le chlorure de fer-III, le chlorure d'étain-II, le chlorure d'étain-IV et le chlorure de fer-III, en particulier le chlorure d'aluminium.

3. Procédé selon la revendication 1 ou 2, dans lequel le catalyseur de Friedel et Crafts est utilisé à raison de 1,0 à 2,5, comme de 1,1 à 1,6, moles par mole de chlorure de 2-chlorothiobenzoyle (A).

4. Procédé selon l'une quelconque des revendications précédentes, dans lequel R¹ et R² sont tous deux des atomes d'hydrogène.

5. Procédé selon l'une quelconque des revendications précédentes. dans lequel R³ est un groupe isopropyle et R⁴ et R⁵ sont tous deux des atomes d'hydrogène.

6. Procédé selon l'une quelconque des revendications précédentes, dans lequel le solvant de Friedel et Crafts est choisi parmi le chlorure de méthylène, le 1,2-dichloroéthane et le 1,1,2,2-tétrachloroéthane.

7. Procédé selon l'une quelconque des revendications précédentes, dans lequel le solvant de Friedel et Crafts est, de plus, présent dans le mélange réactionnel avant que ce dernier soit ajouté à la suspension.

8. Procédé selon la revendication 7, dans lequel les réactifs sont dissous dans un solvant de Friedel et Crafts à raison de 1 à 30 parties en poids par partie en poids du mélange réactionnel.

9. Procédé selon l'une quelconque des revendications précédentes, dans lequel la suspension contient 1 à 20 parties en poids du solvant de Friedel et Crafts par partie en poids du catalyseur.

10. Procédé selon l'une quelconque des revendications précédentes, dans lequel le mélange réactionnel contient de 1 à 20, en particulier de 1,1 à 1,2, moles du composé aromatique (B) par mole du chlorure de 2-chlorothiobenzoyle (A).

11. Procédé selon l'une quelconque des revendications précédentes, mis en oeuvre à une température de 0° à 80°C, en particulier de 20°C à 45°C.

12. Procédé selon l'une quelconque des revendications précédentes, dans lequel le chlorure de 2-chlorothiobenzoyle (A) a été auparavant purifié par distillation sous vide à une température de 80°C à 200°C et sous une pression de 13,3 à 39 997 Pa (0,1 à 300 mm de Hg).

13. Une ou plusieurs thioxanthones préparées par un procédé selon l'une quelconque des revendications précédentes.

14. Mélange cristallin de 2- et de 4-isopropylthioxanthone ayant un point de fusion d'environ 73°C à 74°C.

15. Mélange cristallin de 2- et de 4-chlorothioxanthonc ayant un point de fusion d'environ 143°C à 145°C.

16. Utilisation d'une ou de plusieurs thioxanthoncs selon l'unc quelconque des revendications 12 à 14 pour la préparation d'un produit pharmaceutique à utiliser dans le domaine de la psychothérapie, ou comme activateurs ou sensibilisants dans la photopolymérisation de monomères à insaturation éthylénique.
